# EUROPEAN PATENT SPECIFICATION

(11) **EP 2 123 173 B1**
(45) Date of publication and mention of the grant of the patent: **02.02.2011**
(21) Application number: 07738630.8
(22) Date of filing: 15.03.2007
(51) Int. Cl.: A61K 36/07, A23K 1/18, A23K 1/16, A61P 1/12, A61P 31/04

(54) **METHOD FOR FEEDING MOTHER PIGS WITH GRIFOLA**
VERFAHREN ZUR FÜTTERUNG VON MUTTERSAUEN MIT GRIFOLA
PROCÉDÉ D'ALIMENTATION DE TRUIES AVEC GRIFOLA

(30) Priority: 28.12.2006 JP 2006354443
(43) Date of publication of application: 25.11.2009
(73) Proprietor: Yukiguni Maitake Co., Ltd., Minami-Uonuma-shi, Niigata 949-6695 (JP)
(72) Inventor: SHIGA, Akira, Koyu-gun, Miyazaki 884-0006 (JP); KAMOSHIDA, Akira, Yokohama-shi, Kanagawa 230-0051 (JP); SASA, Yojiro, Minamiuonuma-shi, Niigata 949-7302 (JP); TANIGUCHI, Shin, Minamiuonuma-shi, Niigata 949-6436 (JP); NISHIBORI, Kozo, Tokamachi-shi, Niigata 948-0036 (JP)
(74) Representative: Schwahn, Hartmut
(86) International application number: PCT/JP2007/055180
(87) International publication number: WO 2008/081608

(56) References cited:
- EP-A- 1 155 623
- WO-A-03/043440
- WO-A-2006/119774
- JP-A- 08 038 069
- JP-A- 2001 069 921
- JP-A- 2001 097 881
- JP-A- 2002 153 216
- JP-A- 2003 259 816
- JP-A- 2003 313 139
- JP-B2- 3 328 195
- WU MING-JIUAN ET AL: "Immunomodulatory properties of Grifola frondosa in submerged culture" JOURNAL OF AGRICULTURAL AND FOOD CHEMISTRY, vol. 54, no. 8, April 2006 (2006-04), pages 2906-2914, XP002559526 ISSN: 0021-8561
- WANG ET AL: "Lectins from mushrooms" MYCOLOGICAL RESEARCH, ELSEVIER, GB, vol. 102, no. 8, 1 August 1998 (1998-08-01), pages 897-906, XP022451362 ISSN: 0953-7562
- CHEN ET AL: "Medicinal importance of fungal beta-(1->3), (1->6)-glucans" MYCOLOGICAL RESEARCH, ELSEVIER, GB, vol. 111, no. 6, 1 June 2007 (2007-06-01), pages 635-652, XP022163026 ISSN: 0953-7562

## Description

### TECHNICAL FIELD

The present invention relates to dried *Grifola,* the "Maitake" mushroom (*Grifola frondosa*), dried *Grifola* powder and a *Grifola* extract for use in feeding mother pigs, which maintains good conditions of mother pigs and decreases the number of stillborn piglets and the death rate of piglets in breast-feeding period.

### BACKGROUND ART

In pig-farming industry, the productivity of domestic pigs has seriously decreased due to diseases. As competition in producing domestic animals increases, priority has been put on economical and efficient production. As a result, the comfortable environment for feeding pigs has not been provided. Therefore, various diseases can easily occur in pigs raised under stressful conditions caused by the change in feed technology and the deterioration of the feeding environment. A spate of the pig diseases reduces the farm productivity and gives pig farmers big economic losses. Consequently, it is important to prevent diseases by enhancing the autoimmunity of pigs, in order to increase the productivity in pig farming.

Particularly, the health care of mother pigs greatly influences the productivity. It is considered that improving conditions of mother pigs leads to an increase in the lactation yield in lactation period and gives a great effect to prevent diarrhea in piglets not only in breast-feeding period but also in postweaning period. Additionally, the deterioration of conditions of mother pigs in lactation period leads to a delay in return of estrus and a decrease in the number of piglets to-be-born, and greatly affects the productivity in pig farming.

Since the conditions of mother pigs greatly affect the productivity in pig-farming industry, various studies for improving the productivity in pig farming by maintaining good conditions of mother pigs have been made.

Particularly, feeds and feed additives, which can control conditions of mother pigs, decrease the developing rate of diarrhea and mortality of piglets, and which can increase the number of born piglets, and methods for feeding pigs using the feeds have been proposed. A feed containing galacto-oligosaccharide [JP Patent Publication 1]; a casein digest-containing feed mainly comprising casein phosphopeptide [JP Patent Publication 2]; a method for providing a bacterial cell and a disrupted bacterial cell [JP Patent Publication 3]; a feed containing a guar gum enzymatic decomposition product [JP Patent Publication 4]; a feed using a reduced (activated) folic acid and a method for feeding the same [JP Patent Publications 5 ,JP Patent Publication 6]; a feed for feeding female pigs for breeding containing sugar mainly comprising oligosaccharide [JP Patent Publication 7]; a method for administering a feed mixed with an electrolyte-controlled mineral [JP Patent Publication 8]; a technique characterized by administering transferrin to pregnant mother pigs [JP Patent Publication 9]; and a technique for administering an yeast extract containing ribonucleic acid or 5'-nucleotide [JP Patent Publication 10] have been disclosed.

A technique for feeding a mixed feed for mother pigs has been proposed, the mixed feed being controlled to have 25 to 35% by weight of methionine, 65 to 75% by weight of threonine, 15 to 21% by weight of triptophan, and 83 to 93% by weight of valine, with respect to lysine; containing 10000 to 12000 IU/kg of vitamin A, 1500 to 2500 IU/kg of vitamin D3, 40 to 100 mg/kg of vitamin E and 1 to 3 mg/kg of vitamin K3; or being controlled to have 3200 to 3400 kcal/kg of digestible energy [JP Patent Publication 11].

Additionally, an agent for preventing bacterial infection for animals, by which born piglets can be prevented from diarrhea and skin infection during lactation period when used to treat antepartum mother pigs, has been proposed [JP Patent Publication 12]. A feed additive utilizing excellent properties of Schizophyllum commune culture which is a natural product, and a feed additive characterized by containing a finely ground object of the Schizophyllum commune culture and an yeast have been proposed [JP Patent Publication 13].

However, a technique for increasing the productivity in pig farming by utilizing the "Maitake" mushroom (G*rifola frondosa*) which has been reported as having an immunostimulating property to humans, particularly by applying the same to mother pigs, and for controlling the conditions of mother pigs has not been proposed.

The inventors of the present application have studied about components contained in the "Maitake" mushroom (*Grifola frondosa*) and the use of the same, and found the immunostimulating property of *Grifola* for humans, so as to discover various effects. For example, the inventors have discovered that a fraction obtained by purifying a *Grifola* hydrothermal extract by an alcohol treatment has an immunostimulating property and an antitumor effect [JP Patent Publication 14] and that the fraction usefully effects in influenza virus defense against infection [JP Patent Publication 15], and have filed patent applications.

The inventors of the present application have studied also about application of the "Maitake" mushroom (*Grifola frondosa*) to domestic livestock feeding and have filed a patent application regarding an additive for a livestock feed containing a *Grifola*-derived substance [JP Patent Publication 16]. However, the invention relates to a feed additive for domestic animals, which enables production of meat with high safety and good flavor, and is not particularly intended to maintain good conditions of mother pigs and to decrease the stillborn piglets and the death rate of piglets in breast-feeding period, so as to increase the productivity in pig farming.
JP Patent Publication 1:(Kokai) No. 5-219897 A
JP Patent Publication 2:(Kokai) No. 5-268883 A
JP Patent Publication 3:(Kokai) No. 5-336896 A
JP Patent Publication 4:(Kokai) No. 7-8183 A
JP Patent Publication 5:(Kokai) No. 7-147911 A
JP Patent Publication 6 : (Kokai)No. 8-70788 A
JP Patent Publication 7:(Kokai) No. 8-23889 A
JP Patent Publication 8:(Kokai) No. 10-304825 A
JP Patent Publication 9:(Kokai) No. 11-4637 A
JP Patent Publication 10:(Kokai) No. 2004-313178 A
JP Patent Publication 11:(Kokai) No. 2005-192514 A
JP Patent Publication 12:(Kokai) No. 8-59474 A
JP Patent Publication 13:(Kokai) No. 7-170919 A
JP Patent Publication 14:(Kokai) No. 9-238697 A
JP Patent Publication 15:(Kokai) No. 2005-145934 A
JP Patent Publication 16:(Kokai) No. 2003-259816 A

### DISCLOSURE OF THE INVENTION

### Problems to be Solved by the Invention

An object of the present invention is to maintain good conditions of mother pigs and decrease the stillborn piglets and the death rate of piglets in breast-feeding period, so as to increase the productivity in pig farming.

### Means for Solving the Problems

The inventors have focused on the "Maitake" mushroom (*Grifola frondosa*) reported as having an immunostimulating property and administered dried *Grifola* powder to mother pigs to determine the effect of dried *Grifola* to pigs raising. The inventors discovered that the number of stillborn piglets and the number of dead pigs in breast-feeding period are decreased by administering the powder, and thus have completed the present invention.

That is, the present invention relates to:
(1) a method for feeding pigs, wherein a *Grifola*-derived substance selected from one or more of dried *Grifola,* dried *Grifola* powder and a *Grifola* extract is administered to mother pigs; and
(2) the method for feeding pigs described in (1), wherein mother pigs are postpartum mother pigs.

In the present invention, all types of *Grifola* including "Maitake" (*Grifola frondosa*), "Shiromaitake" (*Grifola albicans)*, "Choreimaitake" (*Dendropolyporus umbellatus*), "Tonbimaitake" (*Grifola gigantea*) and the like can be used. Recently, artificial cultivation of the fruit bodies of "Maitake" (*Grifola frondosa*) has been made possible. It is preferable to use the fruit bodies of *Grifola* in terms of ensuring a stable supply of raw materials.

Fresh *Grifola*, dried *Grifola* or dried *Grifola* powder can be used. The dried powder is easy to handle and preferable. As dried *Grifola*, any *Grifola* prepared by sun drying, hot-air drying or freeze drying can be used.

Dried *Grifola* can be used as it is, or can also be used in the form of small chips, small pieces or fine pieces after appropriate grinding. It is more general to grind dried *Grifola* into powder using a milling apparatus or the like for use, because this leads to a broader application range. In addition, depending on the feeding situation, *Grifola* powder can be appropriately selected from those with large particle sizes to *Grifola* finer powder with small particle sizes.

Dried *Grifola*, dried *Grifola* powder or a *Grifola* extract can be added independently to a feed. To enable thorough mixing of the *Grifola* added to the feed, dried *Grifola*, dried *Grifola* powder or a *Grifola* extract in the dosage form of powders mixed with an extender and a lubricant, granules, pellets, or the like prepared by general methods can also be added to the feed. When a case of using *Grifola* in the form of powder is explained as an example, an extender and a lubricant are added to the dried *Grifola* powder, and then the resultant is thoroughly mixed. At this time, if necessary, substances other than a *Grifola*-derived substance can be added.

As an extender, lactose, starch or dextrin can be used, for example. As a lubricant, a light liquid paraffin can be used. Particularly, when a *Grifola* extract is used, it is preferable to mix the *Grifola* extract with an extender because the *Grifola* extract becomes easily moistened.

Additionally, a medical agent for preventing pig diseases can be used together with dried *Grifola*, dried *Grifola* powder or a *Grifola* extract. As a medical agent, a drug for preventing pneumonia or a disease in digestive system can be fed. Also, a microorganism material including lactobacillus having an immunostimulating effect can be used.

It can be assumed that the method for administering dried *Grifola* according to the present invention to domestic pigs raising improves immunostimulating ability of the pigs. Therefore, the feed can be administered to not only antepartum and postpartum mother pigs but also pigs at any feeding stage with no problem. The method for feeding dried *Grifola* powder according to the present invention provides effects when administered to pigs for a certain period. The feeding period varies depending on the kind of the pig or the feeding stage. However, feeding pigs in the period from 7 days prior to delivery of piglets to weaning of piglets can be a rough idea. It is preferable to give the feed continuously during the period. The feeding amount is preferably around 0.06 g to 0.11 g/kg/day.

In the present invention, "mother pigs" represents, pigs in the period from one week prior to delivery, postpartum pigs, pigs during breast feeding period.

### Effects of the Invention

By administering a *Grifola*-derived substance selected from one or more of dried *Grifola*, dried *Grifola* powder and a *Grifola* extract to mother pigs, good conditions of mother pigs can be maintained and the stillborn piglets and the death rate of piglets in breast-feeding period can decrease, so as to increase the productivity in pig farming.

### BEST MODE FOR CARRYING OUT THE INVENTION

The present invention is described in detail below using Example. However, the present invention is not limited by the Example.

### EXAMPLE 1

### Production of Dried Grifola, Dried Grifola Powder and Grifola Extract

(1) Artificially cultivated fruit bodies of fresh *Grifola frondosa* were lined on the racks of a drying room with racks, and then dried by blowing hot air at approximately 60 °C to approximately 80 °C. By raising the temperature stepwise initially from 60 °C to finally 80 °C for almost one day so as to perform heating and drying, dried fruit bodies of *Grifola frondosa* were obtained. Subsequently, the dried *Grifola frondosa* was powdered with a milling apparatus.

On the other hand, a *Grifola* frondosa extract can be produced with water, alcohol, etc., according to general methods.

(2) At the HS farm, from May to June of 2005, the dried *Grifola frondosa* powder obtained according to the above-described method was fed every day at the ratio of 0.5 % by weight with respect to the weight of the feed, to 80 mother pigs in the period from 7 days prior to delivery of piglets to weaning of piglets.

Next, the number of born piglets from the mother pigs, the number of piglets starting suckling, the number of stillborn piglets, the body weight, the suckling rate, the length of gestation, the number of weaned piglets and the weaning rate were checked every month. Table 1 represents the reference group, and Table 2 represents the example group.

From the results shown in Tables 1 and 2, the average number of stillborn piglets in the example group is 0.56 in comparison with that of the reference group, 1.29. Therefore, it can be seen that the number of stillborn piglets decreased by half in the present invention. That is, it can be recognized that the number of stillborn piglets is decreased by administering dried *Grifola* powder. Additionally, it can be said that the productivity in pig farming increases as the death rate of piglets in breast-feeding period decreases.

The suckling rate, the weaning rate and the final yield of the example group are higher than those of the reference group.

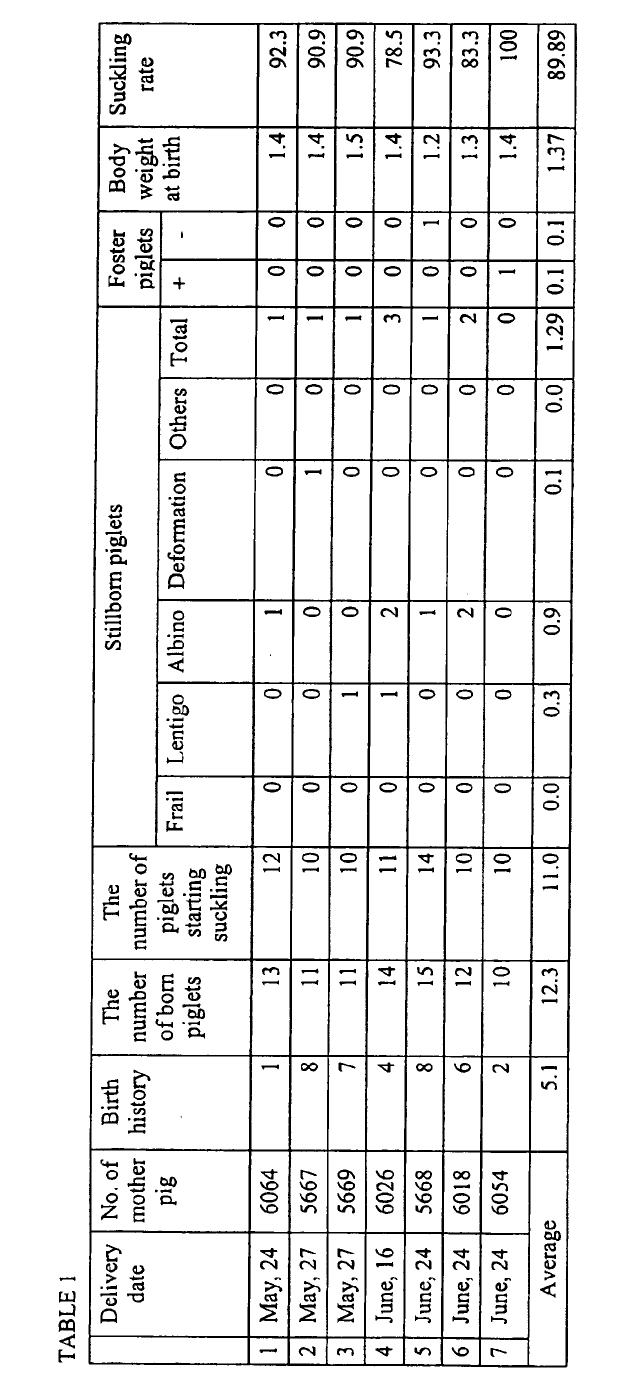

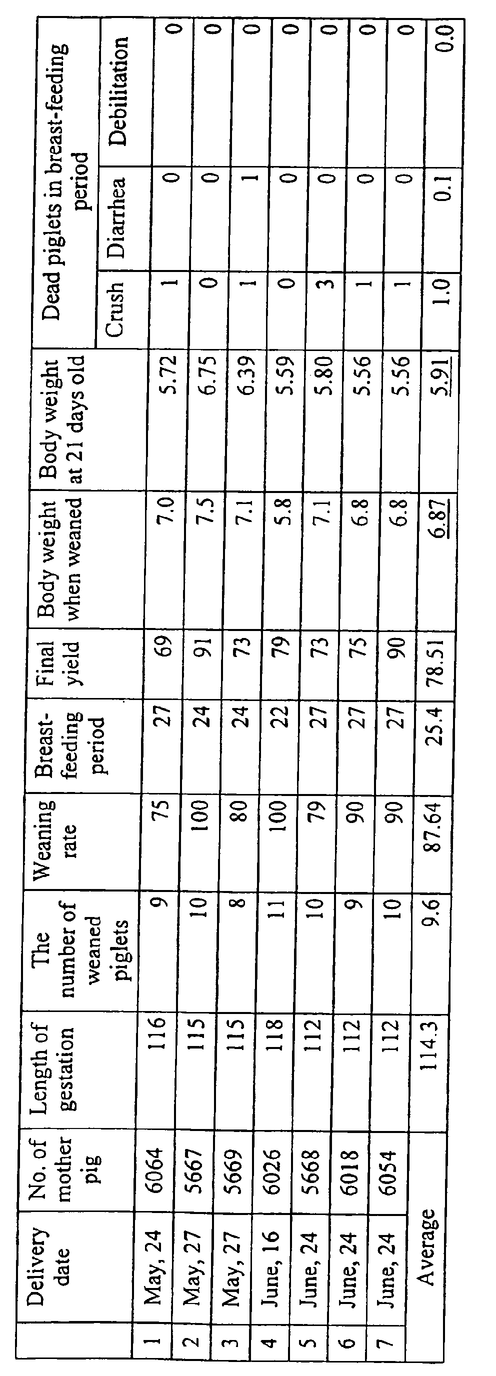

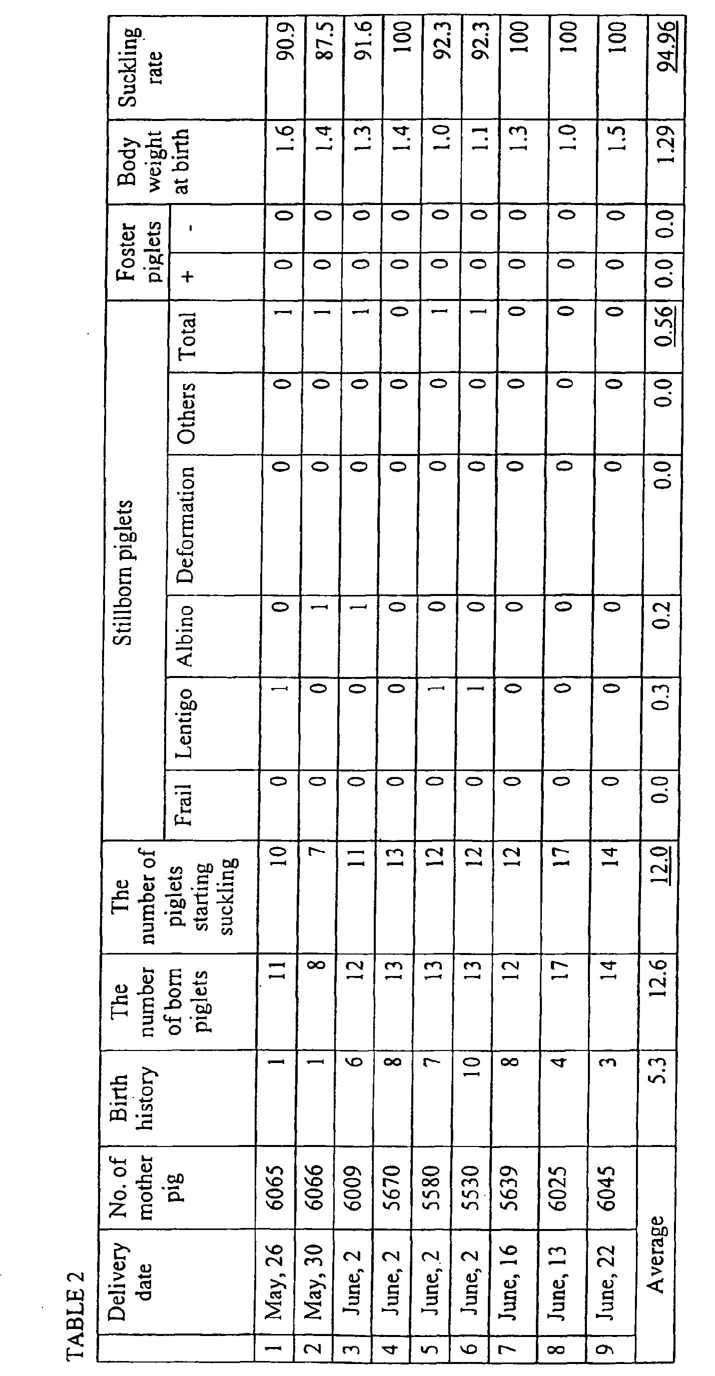

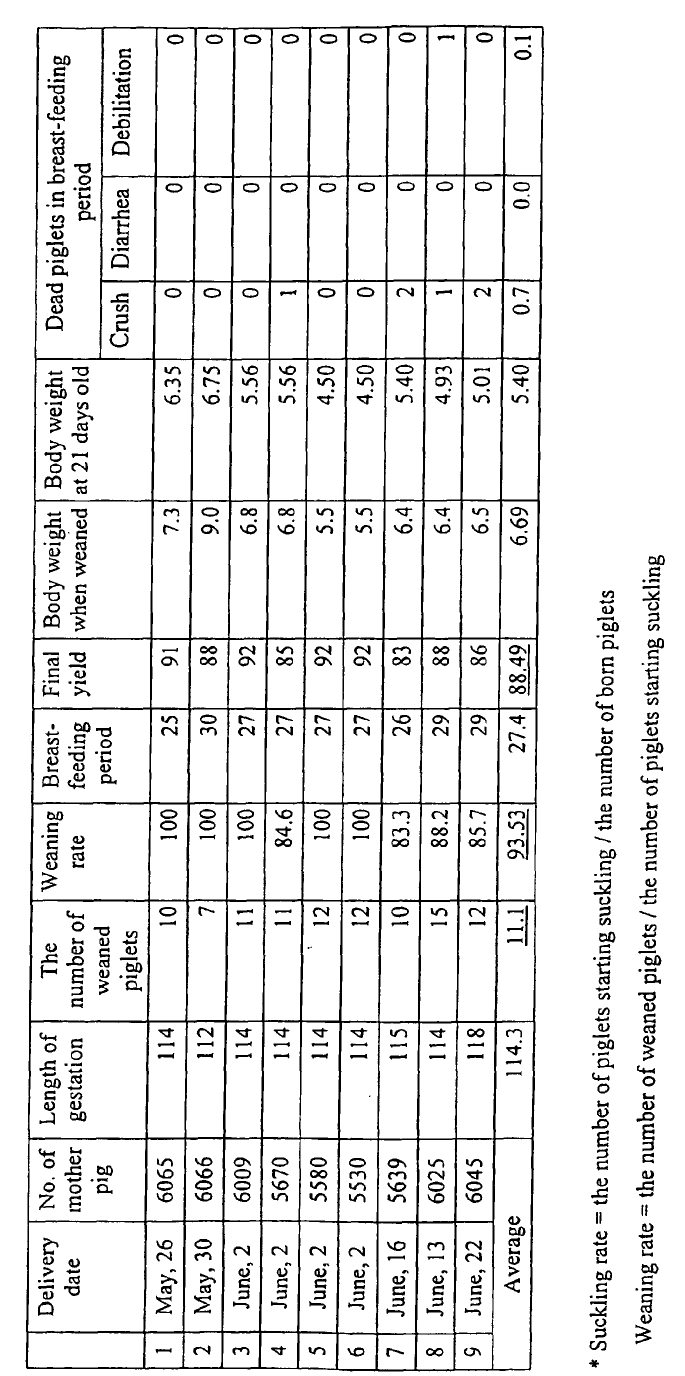

## Claims

1. A *Grifola*-derived substance selected from one or more of dried *Grifola,* dried *Grifola* powder and a *Grifola* extract for use in decreasing the number of stillborn piglets, wherein the *Grifola*-derived substance is administered to mother pigs.

2. A *Grifola*-derived substance selected from one or more of dried *Grifola,* dried *Grifola* powder and a *Grifola* extract for use in decreasing the death rate of piglets during breast feeding period, wherein the *Grifola*-derived substance is administered to mother pigs.

3. A *Grifola*-derived substance for use according to claims 1 or 2, wherein mother pigs are postpartum mother pigs.

## Patentansprüche

1. Von *Grifola* abgeleitete Substanz, ausgewählt aus einem oder mehreren von getrocknetem *Grifola,* Pulver aus getrocknetem *Grifola* und einem *Grifola-*Extrakt, zur Verwendung zur Verringerung der Anzahl von Ferkel-Totgeburten, wobei die von *Grifola* abgeleitete Substanz an die Mutterschweine verabreicht wird.

2. Von *Grifola* abgeleitete Substanz, ausgewählt aus einem oder mehreren von getrocknetem *Grifola,* Pulver aus getrocknetem *Grifola* und einem *Grifola-*Extrakt, zur Verwendung zur Verringerung der Sterblichkeitsrate von Ferkeln während der Säugeperiode, wobei die von *Grifola* abgeleitete Substanz an die Mutterschweine verabreicht wird.

3. Von *Grifola* abgeleitete Substanz zur Verwendung nach Anspruch 1 oder 2, wobei die Mutterschweine post partale Mutterschweine sind.

## Revendications

1. Substance dérivée du *Grifola* sélectionnée parmi un ou plusieurs des éléments *Grifola* séché, poudre de *Grifola* séchée et extrait de *Grifola,* destinés à être utilisés pour réduire le nombre de porcelets mort-nés, la substance dérivée du *Grifola* étant administrée à des truies-mères.

2. Substance dérivée du *Grifola* sélectionnée parmi un ou plusieurs des éléments *Grifola* séché, poudre de *Grifola* séchée et extrait de *Grifola,* destinés à être utilisés pour réduire le taux de mortalité des porcelets lors de la période d'allaitement, la substance dérivée du *Grifola* étant administrée à des truies-mères.

3. Substance dérivée du *Grifola* destinée à être utilisée selon les revendications 1 ou 2, les truies-mères étant des truies-mères post-partum.
